# EUROPEAN PATENT SPECIFICATION

(11) **EP 1 587 489 B1**
(45) Date of publication and mention of the grant of the patent: **03.11.2010**
(21) Application number: 03815790.5
(22) Date of filing: 29.12.2003
(51) Int. Cl.: A61Q 11/00, A61K 8/21

(54) **DENTAL WHITENING COMPOSITIONS AND METHODS**
ZAHNWEISSZUSAMMENSETZUNGEN UND -VERFAHREN
COMPOSITIONS DE BLANCHIMENT DE DENTS ET PROCEDES

(30) Priority: 30.01.2003 US 444039 P; 24.07.2003 US 626142
(43) Date of publication of application: 26.10.2005
(73) Proprietor: 3M Innovative Properties Company, St. Paul, MN 55133-3427 (US)
(72) Inventor: MITRA, Sumita B.,, Saint Paul, MN 55133-3427 (US); BURGIO, Paul A.,, Saint Paul, MN 55133-3427 (US); ALI, Mahfuza B.,, Saint Paul, MN 55133-3427 (US)
(74) Representative: Vossius & Partner
(86) International application number: PCT/US2003/041406
(87) International publication number: WO 2004/069215

(56) References cited:
- EP-A- 0 363 095
- WO-A-01/76549
- WO-A-03/094877
- US-A- 5 607 663

## Description

There is a consumer demand for products and methods for whitening teeth. Although teeth whitening products are available to both dentists and consumers, not all of the products can be conveniently administered using simple and inexpensive equipment Furthermore, some of the products must be repeatedly applied over a sufficient period of time to effect the tooth whitening process. Problems that may lead to inefficient whitening include, for example, temperature increases upon application to the oral surface, which often results in flow from the desired surface, and dilution by saliva.

What is needed are new dental whitening compositions and methods to whiten teeth.

In one aspect, the present invention provides a dental whitening composition suitable for coating oral surfaces comprising:
a tooth whitening agent; and
a polymer comprising:
   a repeating unit comprising a polar or polarizable group; and
   a repeating unit comprising a group selected from the group consisting of a hydrophobic hydrocarbon group having a molecular weight of at least 160, a graft polysiloxane chain, a hydrophobic fluorine-containing group, and combinations thereof,
   with the proviso that the polymer does not include pendant ethylenically unsaturated moieties.

Coatings on hard tissue surfaces of the oral environment of presently disclosed dental whitening compositions are also described herein. As used herein, a "reactive" group is a group that can react under selected conditions (e.g., in the presence of free radicals or under condensation reaction conditions) with another reactive group or another component (e.g., a crosslinker or a compound with condensation reaction sites). For example, in a polymer that includes a reactive group, the reactive group can react with another reactive group and/or another component to form crosslinks through dimerization, oligomerization, and/or polymerization reactions.

As used herein, "repeating unit" or "monomeric unit" refers to a unit in a polymer that is derived from an ethylenically unsaturated monomer. For example, polypropylene includes -CH₂CH(CH₃)- monomeric units that are derived from the ethylenically unsaturated monomer propylene, CH₂=CH(CH₃).

As used herein, "hardenable" refers to a material that can be "hardened". As used herein, "harden" is meant to encompass processes including, for example, crosslinking, dimerization, oligomerization, and/or polymerization reactions.

As used herein, "(meth)acryl" is an abbreviation intended to refer collectively to "acryl" and/or "methacryl".

As used herein, "a", "at least one", and "one or more" are used interchangeably.

As used herein, all numbers are assumed to be modified by the term "about". Also, the recitations of numerical ranges by endpoints include all numbers subsumed within that range (e.g., 1 to 5 includes, for example, 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments.

The present invention provides dental whitening compositions suitable for coating oral surfaces. The dental whitening compositions include a polymer as defined above and a tooth whitening agent The dental whitening composition may optionally include, for example, solvents and additives. Preferably the composition is in the form of a dispersion, suspension, emulsion, or solution. Preferably the composition is an aqueous composition.

The dental whitening compositions may also be hardenable. Hardenable compositions preferably include a reactive polymer and/or a polymerizable component different than the polymer, and an initiator system (e.g., one or more initiators). The reactive polymer and/or polymerizable component may undergo reactions with free radicals. Alternatively, the reactive polymer and/or polymerizable component may undergo condensation reactions including, for example, the presence of moisture. A suitable catalyst to facilitate the condensation reaction may optionally be included. Optionally, an additional compound with condensation reaction sites may also be included to act as a bridging compound between the polymers and/or polymerizable components.

Dental whitening compositions of the present invention may be prepared as a single part liquid or gel by combining the above components. For example, the polymer and the tooth whitening agent may be mixed at the desired temperature (e.g., room temperature). Alternatively, compositions of the present invention may be prepared as multiple part liquids and/or gels that are mixed prior to delivery to the tissue. Such multiple part systems may provide shelf stability that may not exist in single part compositions including, for example, compositions including an initiator system based on two-component redox chemistry, and compositions including an additive that is incompatible with other materials in the composition.

The whitening agent used in the present invention may be any material that has the effect of whitening teeth. Useful whitening agents include, for example, hypochlorites (e.g., sodium hypochlorite), organic peroxides, inorganic peroxides, hydroperoxides, hydrogen peroxide, peracids (also known as peroxyacids), carbamide peroxide (i.e., the urea complex of hydrogen peroxide, CO(NH₂)₂H₂O₂, also known as urea hydrogen peroxide, hydrogen peroxide carbamide, or perhydrol-urea), and combinations thereof. The concentration of a whitening agent in the composition can vary depending, for example, upon its activity.

Preferably, the dental whitening composition includes at least 0.05% by weight, more preferably at least 0.1% by weight, and most preferably at least 0.5% by weight of the tooth whitening agent, based on the total weight of the tooth whitening agent and polymer. Preferably, the dental whitening composition includes at most 50% by weight, more preferably at most 45% by weight, and most preferably at most 40% by weight of the tooth whitening agent, based on the total weight of the tooth whitening agent and polymer.

Polymers disclosed in the present application include a repeating unit that includes a polar or polarizable group as described herein below. Furthermore, the polymers include a repeating unit comprising a group selected from a hydrophobic hydrocarbon group as defined above, a graft polysiloxane chain, a hydrophobic fluorine-containing group, and combinations thereof. In certain embodiments, the polymers also include a repeating unit that includes a fluoride releasing group, a repeating unit that includes a modulating group, or combinations thereof, as described herein below. In some embodiments, the polymer optionally includes a reactive group. Suitable reactive groups (e.g., ethylenically unsaturated groups, epoxy groups, or silane moieties capable of undergoing a condensation reaction) are disclosed, for example, in U.S. Pat. Nos. 5,607,663 (Rozzi et al.), 5,662,887 (Rozzi et al.), 5,866,630 (Mitra et al.), 5,876,208 (Mitra et al.), 5,888,491 (Mitra et al.), and 6,312,668 (Mitra et al.).

The dental whitening composition includes a tooth whitening agent and a polymer including: a repeating unit including a polar or polarizable group; and a repeating unit including a group selected from the group consisting of a hydrophobic hydrocarbon group having a molecular weight of at least 160, a graft polysiloxane chain, a hydrophobic fluorine-containing group, and combinations thereof, with the proviso that the polymer does not include pendant ethylenically unsaturated moieties. Preferably the graft polysiloxane chain has a molecular weight of at least 500. Preferably, the repeating unit including the polar or polarizable group is different than the repeating unit including the group selected from the group consisting of a hydrophobic hydrocarbon group, a graft polysiloxane chain, a hydrophobic fluorine-containing group, and combinations thereof.

Exemplary methods of preparing the recited polymers are well known in the art and include, for example, free radical polymerization conditions as disclosed, for example, in U.S. Pat. Nos. 5,607,663 (Rozzi et al.), 5,662,887 (Rozzi et al.), 5,866,630 (Mitra et al.), 5,876,208 (Mitra et al.), 5,888,491 (Mitra et al.), and 6,312,668 (Mitra et al.).

Dental whitening compositions of the present invention preferably include at least 50% by weight polymer, more preferably at least 55% by weight polymer, and most preferably at least 60% by weight polymer, based on the total weight of the tooth whitening agent and polymer. Dental whitening compositions of the present invention preferably include at most 99.95% by weight polymer, more preferably at most 99.9% by weight polymer, and most preferably at most 99.5% by weight polymer, based on the total weight of the tooth whitening agent and polymer.

Repeating units including a polar or polarizable group are derived from vinylic monomers such as acrylates, methacrylates, crotonates, itaconates, and the like. The polar groups can be acidic, basic or salt. These groups can also be ionic or neutral.

Examples of polar or polarizable groups include neutral groups such as hydroxy, thio, substituted and unsubstituted amido, cyclic ethers (such as oxanes, oxetanes, furans and pyrans), basic groups (such as phosphines and amines, including primary, secondary, tertiary amines), acidic groups (such as oxy acids, and thiooxyacids of C, S, P, B), ionic groups (such as quarternary ammonium, carboxylate salt, sulfonic acid salt and the like), and the precursors and protected forms of these groups. Additionally, a polar or polarizable group could be a macromonomer. More specific examples of such groups follow.

Polar or polarizable groups may be derived from mono- or multifunctional carboxyl group containing molecules represented by the general formula:

CH₂=CR²G-(COOH)_{d}

where R²=H, methyl, ethyl, cyano, carboxy or carboxymethyl, d=1-5 and G is a bond or a hydrocarbyl radical linking group containing from 1-12 carbon atoms of valence d+1 and optionally substituted with and/or interrupted with a substituted or unsubstituted heteroatom (such as O, S, N and P). Optionally, this unit may be provided in its salt form. The preferred monomers in this class are acrylic acid, methacrylic acid, itaconic acid, and N-acryloyl glycine.

Polar or polarizable groups may, for example, be derived from mono- or multifunctional hydroxy group containing molecules represented by the general formula:

CH₂=CR²-CO-L-R³-(OH)_{d}

where R²=H, methyl, ethyl, cyano, carboxy or carboxyalkyl, L=O, NH, d=1-5 and R³ is a hydrocarbyl radical of valence d+1 containing from 1-12 carbon atoms. The preferred monomers in this class are hydroxyethyl (meth)acrylate, hydroxypropyl (meth)acrylate, hydroxybutyl (meth)acrylate, glycerol mono(meth)acrylate, tris(hydroxymethyl)ethane monoacrylate, pentaerythritol mono(meth)acrylate, N-hydroxymethyl (meth)acrylamide, hydroxyethyl (meth)acrylamide, and hydroxypropyl (meth)acrylamide.

Polar or polarizable groups may alternatively be derived from mono- or multifunctional amino group containing molecules of the general formula:

CH₂=CR²-CO-L-R³-(NR⁴R⁵)_{d}

where R², L, R³, and d are as defined above and R⁴ and R⁵ are H or alkyl groups of 1-12 carbon atoms or together they constitute a carbocyclic or heterocyclic group. Preferred monomers of this class are aminoethyl (meth)acrylate, aminopropyl (meth)acrylate, N,N-dimethylaminoethyl (meth)acrylate, N,N-diethylaminoethyl (meth)acrylate, N,N-dimethylaminopropyl (meth)acrylamide, N-isopropylaminopropyl (meth)acrylamide, and 4-methyl-1-acryloyl-piperazine.

Polar or polarizable groups may also be derived from alkoxy substituted (meth)acrylates or (meth)acrylamides such as methoxyethyl (meth)acrylate, 2-(2-ethoxyethoxy)ethyl (meth)acrylate, polyethylene glycol mono(meth)acrylate or polypropylene glycol mono(meth)acrylate.

Polar or polarizable groups units may be derived from substituted or unsubstituted ammonium monomers of the general formula: 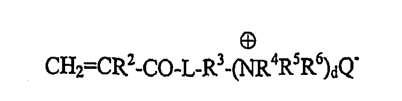 where R², R³, R⁴, R⁵, L and d are as defined above, and where R⁶ is H or alkyl of 1-12 carbon atoms and Q⁻ is an organic or inorganic anion. Preferred examples of such monomers include 2-N,N,N-trimethylammonium ethyl (meth)acrylate, 2-N,N,N-triethylammonium ethyl (meth)acrylate, 3-N,N,N-trimethylammonium propyl (meth)acrylate, N-(2-N',N',N'-trimethylammonium) ethyl (meth)acrylamide, N-(dimethyl hydroxyethyl ammonium) propyl (meth)acrylamide, or combinations thereof, where the counterion may include fluoride, chloride, bromide, acetate, propionate, laurate, palmitate, stearate, or combinations thereof. The monomer can also be N,N-dimethyl diallyl ammonium salt of an organic or inorganic counterion.

Ammonium group containing polymers can also be prepared by using as the polar or polarizable group any of the amino group containing monomer described above, and acidifying the resultant polymers with organic or inorganic acid to a pH where the pendant amino groups are substantially protonated. Totally substituted ammonium group containing polymers may be prepared by alkylating the above described amino polymers with alkylating groups, the method being commonly known in the art as the Menschutkin reaction.

Polar or polarizable groups can also be derived from sulfonic acid group containing monomers, such as vinyl sulfonic acid, styrene sulfonic acid, 2-acrylamido-2-methyl propane sulfonic acid, allyloxybenzene sulfonic acid, and the like. Alternatively, polar or polarizable groups may be derived from phosphorous acid or boron acid group-containing monomers. These monomers may be used in the protonated acid form as monomers and the corresponding polymers obtained may be neutralized with an organic or inorganic base to give the salt form of the polymers.

Exemplary polar or polarizable groups are disclosed, for example, in U.S. Pat. Nos. 5,607,663 (Rozzi et al.), 5,662,887 (Rozzi et al.), 5,866,630 (Mitra et al.), 5,876,208 (Mitra et al.), 5,888,491 (Mitra et al.), and 6,312,668 (Mitra et al.).

Preferred repeating units of a polar or polarizable group include acrylic acid, itaconic acid, N-isopropylacrylamide, or combinations thereof.

Suitable fluoride releasing groups include fluoride salts as disclosed, for example, in U.S. Pat. Nos. 5,607,663 (Rozzi et al.), 5,662,887 (Rozzi et al.), 5,866,630 (Mitra et al.), 5,876,208 (Mitra et al.), 5,888,491 (Mitra et al.), and 6,312,668 (Mitra et al.). A preferred fluoride releasing group includes tetrafluoroborate anions as disclosed, for example, in U.S. Pat. No. 4,871,786 (Aasen et al.). A preferred repeating-unit of a fluoride releasing group includes trimethylammoniumethyl methacrylate.

An examplary hydrophobic hydrocarbon group is derived from an ethylenically unsaturated preformed hydrocarbon moiety having a weight average molecular weight greater than 160. The hydrocarbon moiety has a molecular weight of at least 160. Preferably the hydrocarbon moiety has a molecular weight of at most 100,000, and more preferably at most 20,000. The hydrocarbon moiety may be aromatic or non-aromatic in nature, and optionally may contain partially or fully saturated rings. Preferred hydrophobic hydrocarbon moieties are dodecyl and octadecyl acrylates and methacrylates. Other preferred hydrophobic hydrocarbon moieties include macromonomers of the desired molecular weights prepared from polymerizable hydrocarbons, such as ethylene, styrene, alpha-methyl styrene, vinyltoluene, and methyl methacrylate.

Exemplary hydrophobic hydrocarbon groups are disclosed, for example, in U.S. Pat. Nos. 5,607,663 (Rozzi et al.), 5,662,887 (Rozzi et al.), 5,866,630 (Mitra et al.), 5,876,208 (Mitra et al.), 5,888,491 (Mitra et al.), and 6,312,668 (Mitra et al.).

Exemplary repeating units of hydrophobic fluorine-containing groups include acrylic or methacrylic acid esters of 1,1-dihydroperfluoroalkanols and homologs: CF₃(CF₂)ₓCH₂ OH and CF₃(CF₂)ₓ(CH₂)_{y}OH, where x is zero to 20 and y is at least 1 up to 10; ω-hydrofluoroalkanols (HCF₂(CF₂)ₓ(CH₂)_{y}OH), where x is 0 to 20 and y is at least 1 up to 10; fluoroalkylsulfonamido alcohols; cyclic fluoroalkyl alcohols; and CF₃(CF₂CF₂O)_{q}(CF₂O)ₓ(CH₂)_{y}OH, where q is 2 to 20 and greater than x, x is 0 to 20, and y is at least 1 up to 10.

Preferred repeating units of a hydrophobic fluorine-containing group include 2-(methyl(nonafluorobutyl)sulfonyl)amino)ethyl acrylate, 2-(methyl(nonafluorobutyl)sulfonyl)amino)ethyl methacrylate, or combinations thereof.

Exemplary hydrophobic fluorine-containing groups are disclosed, for example, in U.S. Pat. Nos. 5,607,663 (Rozzi et al.), 5,662,887 (Rozzi et al.), 5,866,630 (Mitra et al.), 5,876,208 (Mitra et al.), 5,888,491 (Mitra et al.), and 6,312,668 (Mitra et al.).

The graft polysiloxane chain is derived from an ethylenically unsaturated preformed organosiloxane chain. The molecular weight of this unit is generally above 500. Preferred repeating units of a graft polysiloxane chain include a silicone macromer.

Monomers used to provide the graft polysiloxane chain of this invention are terminally functional polymers having a single functional group (vinyl, ethylenically unsaturated, acryloyl, or methacryloyl group) and are sometimes termed macromonomers or "macromers". Such monomers are known and may be prepared by methods as disclosed, for example, in U.S. Pat. Nos. 3,786,116 (Milkovich et al.) and 3,842,059 (Milkovich et al.). The preparation of polydimethylsiloxane macromonomer and subsequent copolymerization with vinyl monomer have been described in several papers by Y. Yamashita et al., [Polymer J. 14, 913 (1982); ACS Polymer Preprints 25 (1), 245 (1984); Makromol. Chem. 185, 9 (1984)].

Exemplary polysiloxane chains are disclosed, for example, in U.S. Pat. Nos. 5,468,477 (Kumar et al.), 5,607,663 (Rozzi et al.), 5,662,887 (Rozzi et al.), 5,725,882 (Kumar et al.), 5,866,630 (Mitra et al.), 5,876,208 (Mitra et al.), 5,888,491 (Mitra et al.), and 6,312,668 (Mitra et al.).

Exemplary modulating groups are derived from acrylate or methacrylate or other vinyl polymerizable starting monomers and optionally contain functionalities that modulate properties such as glass transition temperature, solubility in the carrier medium, hydrophilic-hydrophobic balance and the like.

Examples of modulating groups include the lower to intermediate methacrylic acid esters of 1-12 carbon straight, branched or cyclic alcohols. Other examples of modulating groups include styrene, vinyl esters, vinyl chloride, vinylidene chloride, acryloyl monomers and the like.

Additional exemplary modulating groups are disclosed, for example, in U.S. Pat. Nos. 5,607,663 (Rozzi et al.), 5,662,887 (Rozzi et al.), 5,866,630 (Mitra et al.), 5,876,208 (Mitra et al.), 5,888,491 (Mitra et al.), and 6,312,668 (Mitra et al.).

Dental whitening compositions of the present invention optionally include an initiator system or catalyst that enables the composition to be hardened. For example, visible and/or near-infrared photoinitiator systems may be used to initiate photopolymerization in compositions including free-radically polymerizable components. For example, a monomer can be combined with a three component or ternary photoinitiator system including a sensitizer, an electron donor, and an iodonium salt as disclosed, for example, in U.S. Pat No. 5,545,676 (Palazzotto et al.). Alternatively, the composition may include a binary initiator system including a sensitizer (e.g., camphorquinone) and an electron donor (e.g., a secondary or a tertiary alkyl amine compound as disclosed, for example, in U.S. Pat. No. 4,071,424 (Dart et al.)).

Another class of useful photoinitiators includes acylphosphine oxides, as disclosed in European Pat. Publ. No. 173,567 (Ying). Such acylphosphine oxides are of the general formula (R)₂P(=O)C(=O)-R¹, wherein each R individually can be a hydrocarbyl group (e.g., alkyl, cycloalkyl, aryl, and aralkyl), which may be substituted with a halo-, alkyl- or alkoxy-group, or the two R groups may be joined to form a ring along with the phosphorous atom, and wherein R¹ is a hydrocarbyl group, an S-, O-, or N-containing five- or six-membered heterocyclic group, or a -Z-C(=O)-P(=O)-(R)₂ group, wherein Z represents a divalent hydrocarbyl group (e.g., alkylene or phenylene) having from 2 to 6 carbon atoms.

Preferred acylphosphine oxides useful in the invention are those in which the R and R¹ groups are phenyl or lower alkyl- or lower alkoxy-substituted phenyl. By "lower alkyl" and "lower alkoxy" is meant such groups having from 1 to 4 carbon atoms. Most preferably, the acylphosphine oxide is bis(2,4,6-trimethylbenzoyl)phenyl phosphine oxide available under the trade designation IRGACURE 819 from Ciba Specialty Chemicals (Tarrytown, NY).

The use of redox catalysts including oxidants and reductants for inducing free radical polymerization in multi-component systems is also useful for generating hardened gels. A preferred mode of initiating the polymerization reaction uses oxidizing and reducing agents as a redox catalyst system. Various redox systems optionally including microencapsulated reducing and/or oxidizing agents are disclosed in U.S. Pat. No. 5,154,762 (Mitra et al.).

Preferably, the oxidizing agent reacts with or otherwise cooperates with the reducing agent to produce free radicals. The free radicals are capable of initiating polymerization of the ethylenically unsaturated moiety. The oxidizing and reducing agents preferably are sufficiently soluble and are present in an amount sufficient to permit an adequate free radical reaction rate as disclosed in U.S. Pat. No. 6,136,885 (Rusin et al.).

A preferred class of oxidizing agents includes persulfates (e.g., sodium, potassium, ammonium, and alkyl ammonium persulfates). Another preferred class of oxidizing agents includes peroxides or peroxide salts (e.g., hydrogen peroxide, benzoyl peroxide, and hydroperoxides including, for example cumene hydroperoxide, tert-butyl hydroperoxide, tert-amyl hydroperoxide, and 2,5-dihydroperoxy-2,5-dimethylhexane). Other preferred oxidizing agents include salts of cobalt (III) and iron (III), perboric acid and its salts, and salts of a permanganate anion. Combinations of any of the above mentioned oxidizing agents can also be used.

Preferred reducing agents include, for example, amines (e.g., aromatic amines), ascorbic acid, metal complexed ascorbic acid, cobalt (II) chloride, ferrous chloride, ferrous sulfate, hydrazine, hydroxylamine, oxalic acid, thiourea, and salts of dithionite, thiosulfate, benzene sulfinate, or sulfite anions.

If initiators are included in compositions of the present invention, the compositions preferably include at least 0.01% by weight of the initiator and more preferably at least 0.1% by weight of the initiator, based on the total weight of the composition. If initiators are included in compositions of the present invention, the compositions preferably include at most 10% by weight of the initiator and more preferably at most 5% by weight of the initiator, based on the total weight of the composition.

Hardenable dental whitening compositions of the present invention preferably include a reactive polymer and/or a polymerizable component different than the polymer (e.g., a secondary polymerizable component).

In certain embodiments, the compositions are photopolymerizable, i.e., the compositions contain a photoinitiator (i.e., a photoinitiator system) that upon irradiation with actinic radiation initiates the polymerization (or hardening) of the composition. Such photopolymerizable compositions are preferably free radically polymerizable.

In certain embodiments, the compositions are chemically polymerizable, i.e., the compositions contain a chemical initiator system that can polymerize, cure, or otherwise harden the composition without dependence on irradiation with actinic radiation. Such chemically polymerizable compositions are sometimes referred to as "self-cure" compositions and may include, for example, glass ionomer cements (e.g., conventional and resin-modified glass ionomer cements), redox cure systems, silane moieties capable of undergoing a condensation reaction (as described herein above), and combinations thereof.

Ethylenically unsaturated compounds include, for example, polymerizable monomers, polymerizable oligomers, polymerizable polymers, and combinations thereof. Preferably, the polymerizable component is free radically polymerizable. Preferred monomers, oligomers, and polymers are those which are partially or fully water miscible.

Suitable polymerizable monomers and oligomers include, for example, poly(ethyleneglycol) dimethacrylate (PEGDMA), tetrahydrofurfural methacrylate, as well as hydroxylic functional monomers including, for example, 2-hydroxyethyl methacrylate (HEMA), glycidyl dimethacrylate (GDMA), and glycidyl monomethacrylate (GMMA). Hydrophobic monomers and oligomers including, for example, bis(glycidyl methacrylate) (bis-GMA), tri(ethyleneglycol) dimethacrylate (TEGDMA), and urethane dimethacrylate may also be utilized.

Suitable polymerizable polymers include, for example, partially or fully acrylate- or methacrylate-functionalized polymers including, for example, functionalized poly(acrylic acid) polymers, cellulosics, poly(vinylalcohol) polymers, poly(oxyethylene)-poly(oxypropylene) block copolymers, poly(ethyleneglycol) polymers, and the like.

Chemically polymerizable compositions may include glass ionomer cements such as conventional glass ionomer cements that typically employ as their main ingredients a homopolymer or copolymer of an ethylenically unsaturated carboxylic acid (e.g., poly acrylic acid, copoly (acrylic, itaconic acid), and the like), a fluoroaluminosilicate ("FAS") glass, water, and a chelating agent such as tartaric acid. Conventional glass ionomers (i.e., glass ionomer cements) typically are supplied in powder/liquid formulations that are mixed just before use. The mixture will undergo self-hardening in the dark due to an ionic reaction between the acidic repeating units of the polycarboxylic acid and cations leached from the glass. The glass ionomer cements may also include resin-modified glass ionomer ("RMGI") cements. Exemplary chemically polymerizable compositions are described, for example, in US 2004/0120901.

The chemically polymerizable compositions may include redox cure systems that include a polymerizable component (e.g., an ethylenically unsaturated polymerizable component) and redox agents. The redox agents may include an oxidizing agent and a reducing agent. Suitable polymerizable components, redox agents, optional acid-functional components, and optional fillers that are useful in the present invention are described in Applicants' Assignees' copending Publication No. 2003-0166740, published September 4, 2003 and Publication No. 2003-0195273, published October 16, 2003.

The reducing and oxidizing agents should react with or otherwise cooperate with one another to produce free-radicals capable of initiating polymerization of the resin system (e.g., the ethylenically unsaturated component). This type of cure is a dark reaction, that is, it is not dependent on the presence of light and can proceed in the absence of light. The reducing and oxidizing agents are preferably sufficiently shelf-stable and free of undesirable colorization to permit their storage and use under typical dental conditions. They should be sufficiently miscible with the resin system (and preferably water-miscible) to permit ready dissolution in (and discourage separation from) the other components of the polymerizable composition.

Useful reducing agents include ascorbic acid, ascorbic acid derivatives, and metal complexed ascorbic acid compounds as described in U.S. Pat. No. 5,501,727 (Wang et al.); amines, especially tertiary amines, such as 4-tert-butyl dimethylaniline; aromatic sulfinic salts, such as p-toluenesulfinic salts and benzenesulfinic salts; thioureas, such as 1-ethyl-2-thiourea, tetraethyl thiourea, tetramethyl thiourea, 1,1-dibutyl thiourea, and 1,3-dibutyl thiourea; and mixtures thereof. Other secondary reducing agents may include cobalt (II) chloride, ferrous chloride, ferrous sulfate, hydrazine, hydroxylamine (depending on the choice of oxidizing agent), salts of a dithionite or sulfite anion, and mixtures thereof. Preferably, the reducing agent is an amine.

Suitable oxidizing agents will also be familiar to those skilled in the art, and include but are not limited to persulfuric acid and salts thereof, such as sodium, potassium, ammonium, cesium, and alkyl ammonium salts. Additional oxidizing agents include peroxides such as benzoyl peroxides, hydroperoxides such as cumyl hydroperoxide, t-butyl hydroperoxide, and amyl hydroperoxide, as well as salts of transition metals such as cobalt (III) chloride and ferric chloride, cerium (IV) sulfate, perboric acid and salts thereof, permanganic acid and salts thereof, perphosphoric acid and salts thereof, and mixtures thereof.

It may be desirable to use more than one oxidizing agent or more than one reducing agent. Small quantities of transition metal compounds may also be added to accelerate the rate of redox cure. In some embodiments it may be preferred to include a secondary ionic salt to enhance the stability of the polymerizable composition as described in Applicants' copending Publication No. 2003-0195273, published October 16, 2003.

The reducing and oxidizing agents are present in amounts sufficient to permit an adequate free-radical reaction rate. This can be evaluated by combining all of the ingredients of the polymerizable composition except for the optional filler, and observing whether or not a hardened mass is obtained.

The reducing or oxidizing agents can be microencapsulated as described in U.S. Pat. No. 5,154,762 (Mitra et al.). This will generally enhance shelf stability of the polymerizable composition, and if necessary permit packaging the reducing and oxidizing agents together. For example, through appropriate selection of an encapsulant, the oxidizing and reducing agents can be combined with an acid-functional component and optional filler and kept in a storage-stable state. Likewise, through appropriate selection of a water-immiscible encapsulant, the reducing and oxidizing agents can be combined with an FAS glass and water and maintained in a storage-stable state.

A redox cure system can be combined with other cure systems, e.g., with a glass ionomer cement and with a photopolymerizable composition such as described U.S. Pat. No. 5,154,762 (Mitra et al.).

The hardenable compositions that utilize a redox cure system can be supplied in a variety of forms including two-part powder/liquid, paste/liquid, and paste/paste systems. Other forms employing multi-part combinations (i.e., combinations of two or more parts), each of which is in the form of a powder, liquid, gel, or paste are also possible. In a multi-part system, one part typically contains the reducing agent(s) and another part typically contains the oxidizing agent(s). Therefore, if the reducing agent is present in one part of the system, then the oxidizing agent is typically present in another part of the system. However, the reducing agent and oxidizing agent can be combined in the same part of the system through the use of the microencapsulation technique.

In some embodiments, compositions of the present invention include, or may optionally include, additives (e.g., medical additives for medical compositions that are suitable for use in or on the body, dental additives for dental compositions that are suitable for use in the oral environment). Exemplary additives include, for example, fluoride sources, anticaries agents (e.g., xylitol), remineralizing agents (e.g., calcium phosphate compounds), breath fresheners, anesthetics, clotting agents, acid neutralizers, chemotherapeutic agents, immune response modifiers, medicaments, indicators, dyes, pigments, wetting agents, surfactants, buffering agents, viscosity modifiers, thixotropes, fillers, polyols, antimicrobial agents, antifungal agents, stabilizers, agents for treating xerostomia, and combinations thereof. Preferably the additives are dental additives suitable for use in the oral environment

Useful additives may be selected for specific applications as desired. Dental whitening compositions of the present invention may be adjusted as desired to include the amount of additive as desired for the specific application.

The dental whitening compostions of the present invention are preferably applied from a solvent. Useful solvents include, for example, alcohols, glycols, poly(oxyalkylene) glycols, water, and combinations thereof.

Dental whitening compositions of the present invention may also include non-polymerizable polymers. Preferably, the non-polymerizable polymers are partially or fully miscible in an aqueous environment and include, for example, poly(acrylic acid) polymers, cellulosics, poly(vinylalcohol) polymers, poly(oxyethylene)-poly(oxypropylene) block copolymers, poly(ethyleneglycol) polymers, and combinations thereof.

Methods of the present invention provide for the treatment of soft and hard tissues, including human and animal tissues. Hard tissues include, for example, bone, teeth, and the component parts of teeth (e.g., enamel, dentin, and cementum). Soft tissues include, for example, mucosa (e.g., tongue, gingiva, and throat).

Dental whitening compositions of the present invention may be delivered to the desired site by any method as desired. For example, the composition may be delivered directly onto the tissue from a container or dispenser. Suitable containers or dispensers include, for example, bottles, vials, syringes, and tubes. The ability to delivery the composition as a bulk liquid from a needle tip or as a fine mist from an aerosol provides versatility in application. Alternatively, the composition can be delivered by using a brush, sponge, applicator, or swab to paint or coat the composition onto the tissue. For some applications it may be desirable to apply the composition to larger areas. For those particular applications, the compositions may be delivered via spray or aerosol dispensers or by simply rinsing the entire tissue area (e.g., the oral cavity) with the liquid. Another alternative mode of delivery includes the use of a tray type dispenser.

Alternatively, the composition can be applied to a substrate, and the
substrate having the composition thereon can be applied to the desired surface. Suitable substrates include, for example, polymeric films, paper, and woven and non-woven sheets. The composition can also be applied to a brush, spatula, medical/dental instrument, or an applicator prior to application to the desired surface.

When the dental whitening compositions of the present invention include two or more parts, the two or more parts are preferably mixed just prior to or during the application process. Suitable mixing devices include, for example, static mixing devices.

The composition are preferably allowed to stand on the surface of the tissue long enough to provide the desired effect. The standing time will vary depending on the particular composition employed, the type of tissue, the intended use, and the time available for carrying out the procedure. For many applications, the composition may be allowed to remain on the tissue for an extended period of time.

For some embodiments of the present invention, dental whitening compositions may be hardened, for example, by inducing a reactive polymer to react. If the dental whitening composition includes an optional polymerizable component different than the reactive polymer, hardening of the composition may also include polymerization of the polymerizable component. For example, when the reactive polymer or the polymerizable component includes an ethylenically unsaturated group, polymerization may be induced by the application of actinic radiation. Preferably the composition is irradiated with radiation having a wavelength of 400 to 1200 nanometers, and more preferably with visible radiation. Visible light sources include, for example, the sun, lasers, metal vapor (e.g., sodium and mercury) lamps, incandescent lamps, halogen lamps, mercury arc lamps, fluorescent room light, flashlights, light emitting diodes, tungsten halogen lamps, and xenon flash lamps.

Alternatively, for embodiments of the present invention in which the reactive polymer or the polymerizable component includes an ethylenically unsaturated group, the composition may include two or more parts, with one part including an oxidizing agent, and another part including a reducing agent.

Once a composition of the present invention has been hardened, the composition is generally not readily removed. However, the hardened composition can generally be removed by mechanical or chemical methods including, for example, brushing, wiping, scraping, and use of solvents (e.g., alcohols).

Objects and advantages of this invention are further illustrated by the following examples, but the particular materials and amounts thereof recited in these examples, as well as other conditions and details, should not be construed to unduly limit this invention. Unless otherwise indicated, all parts and percentages are on a weight basis, all water is deionized water, and all molecular weights are weight average molecular weight

### EXAMPLES

### Abbreviations/Definitions

| | |
|---|---|
| NIPAAM | N-Isopropyl acrylamide (Sigma-Aldrich, St. Louis, MO) |
| IEM | 2-Isocyanatoethyl methacrylate (Sigma-Aldrich) |
| AA | Acrylic acid (Sigma-Aldrich) |
| MAA | Methacrylic acid (Sigma-Aldrich) |
| ITA | Itaconic acid (Sigma-Aldrich) |
| NVP | N-Vinyl Pyrrolidone (Sigma-Aldrich) |
| LA | Lauryl acrylate (Signa-Aldrich) |
| IBMA | Isobutyl methacrylate (Sigma-Aldrich) |
| ODA | Octadecyl acrylate (Sigma-Aldrich) |
| A-174 | 3-(Trimethoxysilyl)propyl methacrylate (Sigma-Aldrich) |
| SiMac | Silicone macromer of MW about 10,000 (prepared as described for making "monomer C 3b" at column 16 of U.S. Pat. No. 4,693,935 (Mazurek)) |
| TMA-BF₄ | Trimethylammoniumethyl methacrylate tetrafluoroborate (Prepared as described for SM-1) |
| DMA-C₁₆Br | Dimethylhexadecylammoniumethyl methacrylate bromide (Prepared as described for SM-2) |
| MeFBSEA | 2-(Methyl(nonafluorobutyl)sulfonyl)amino)ethyl acrylate (Prepared as described in Example 2 of International Publication No. WO 01/30873 (Savu et al.)) |
| MeFBSEMA | 2-(Methyl(nonafluorobutyl)sulfonyl)amino)ethyl methacrylate (Prepared as described in Example 2 of International Publication No. WO 01/30873 (Savu et al.)) |
| DMAEMA | Dimethylaminoethyl methacrylate (Sigma-Aldrich) |
| F127 | PLURONIC F127 Polyoxyethylene-polyoxypropylene block copolymer (BASF Wyandotte, Wyandotte, MI) |
| VAZO-67 | 2,2'-Azobis(2-methylbutanenitrile) (Dupont, Wilmington, DE) |
| V-50 | 2,2'-Azobis(2-amidinopropane) dihydrochloride (WAKO, Richmond, VA) |
| BHT | 2,6-Di-tert-butyl-4-methylphenol (Sigma-Aldrich) |
| DBTDL | Dibutyltin dilaurate (Sigma-Aldrich) |
| THF | Tetrahydrofuran (Sigma-Aldrich) |
| DMF | Dimethyl formamide (Sigma-Aldrich) |

### STARTING MATERIALS

### SM-1: Synthesis of Trimethylammoniumethyl Methacrylate Tetrafluoroborate (TMA-BF₄)

A three-necked flask fitted with a mechanical stirrer, a dropping funnel and a condenser was charged with 80 parts of sodium tetrafluoroborate (Alfa Aesar Inorganics, Ward Hill, MA) and 130 parts of DI water. The mixture was stirred for 15 minutes and a clear solution was obtained. From the dropping funnel a solution of 202.4 parts of dimethylaminoethyl methacrylate-methyl chloride (trimethylammoniumethyl methacrylate chloride; CPS Company, Ciba, Crystal Lake, IL) and 80 parts of DI water was added slowly. A solid product immediately began to precipitate out. After the addition was complete, the mixture was stirred for 30 minutes and the solid isolated by filtration, washed with 30 parts of DI water, and dried under vacuum at 40°C. An NMR analysis of the solid product revealed the structure to be pure trimethylammoniumethyl methacrylate tetrafluoroborate.

### SM-2: Syntheses of Dimethylhexadecylammoniumethyl Methacrylate Bromide (DMA-C₁₆Br)

A.500-ml round-bottom flask was charged with 42.2 parts of DMAEMA, 154.7 parts of acetone, 93.2 parts of 1-bromohexadecane (Sigma-Aldrich), and 0.34 parts of BHT. The mixture was stirred for 16 hours at 35°C and then allowed to cool to room temperature. The resulting white solid precipitate was isolated by filtration, washed with cold ethyl acetate, and dried under vacuum at 40°C. An NMR analysis of the solid product revealed the structure to be pure dimethylhexadecylammoniumethyl methacrylate bromide.

### EXAMPLE 1 (Reference example)

### Preparation of Poly(AA(40)/ITA(20)/NIPAAAM(20)/TMA-BF₄(20)) in THF

AA (40 parts), ITA (20 parts), NIPAAM (20 parts), TMA-BF₄ (20 parts), VAZO-67 (1.0 part), and THF (300 parts) were combined in a reaction vessel and the resulting mixture purged with nitrogen for 2 minutes. The vessel was sealed and maintained at 65°C in a constant temperature rotating device for 18 hours during which time a white solid precipitate formed. After cooling to room temperature, water (300 parts) was added to the mixture with agitation until the solid was dissolved. The resulting polymer solution was designated Example 1 and identified as the polymer of AA (40), ITA (20), NIPAAM (20), and TMA-BF₄ (20) with weight ratios indicated in parentheses. The polymer solution was determined to have 18.4% solids.

### EXAMPLES 2-8

### Preparation of NIPAAM Containing Polymers in THF Solvent

Polymer solutions designated Examples 2-8 were prepared as described for Example 1 and are listed as follows with monomeric units, weight ratios, and % Solids indicated:

| | |
|---|---|
| Example 2: | AA(40)/ITA(20)/NIPAAM(20)/TMA-BF₄(18)/LA(2); 21.7% Solids |
| Example 3: | AA(40)/ITA(20)/NIPAAM(20)/TMA-BF₄(18)/SiMac(2); 19.2% Solids |
| Example 4: | AA(40)/ITA(20)/NIPAAM(20)/TMA-BF₄(18)/ODA(2); 18.8% Solids |
| Example 5: | AA(40)/ITA(20)/NIPAAM(20)/TMA-BF₄(19)/SiMac(1); 25.7% Solids |
| Example 6*: | AA(40)/ITA(17)/NIPAAM(20)/TMA-BF₄(18)/ODA(2)/ A-174(3); 20.0% Solids |
| Example 7: | AA(40)/ITA(20)/MeFBSEA(1)/NIPAAM(20)/TMA-BF₄(19); 15.3% Solids |
| Example 8: | AA(40)/ITA(20)/MeFBSEA(1)/NIPAAM(20)/TMA-BF₄(19); 13.3% Solids |

| | |
|---|---|
| *Example 6 polymer solution was made in a 1:1 THF-ethanol solvent and no water was added after polymerization. | |

### EXAMPLE 9

### Preparation of Poly(AA(40)/ITA(20)/NIPAAM(20)/TMA-BF₄(18/SiMac(2)) in Water

AA (40 parts), ITA (20 parts), NIPAAM (20 parts), TMA-BF₄ (18 parts), SiMac (2 parts), V-50 (1 part), and DI water (300 parts) were combined in a reaction vessel and the resulting mixture purged with nitrogen for 5 minutes. The vessel was sealed and maintained at 60°C in a constant temperature rotating device for 30 hours. Upon cooling to room temperature a viscous polymer solution was obtained that was designated Example 9 and identified as the polymer of AA (40), ITA (20), NIPAAM (20), TMA-BF₄(20), and SiMac (2 parts) with weight ratios indicated in parentheses. The polymer solution was determined to have 20.0% solids.

### EXAMPLES 10-13

### Preparation of Various Polymers in Water

Polymer solutions designated Examples 10-13 were prepared as described for Example 9 and are listed as follows with monomeric units, weight ratios, and % Solids indicated:

| | |
|---|---|
| Example 10: | AA(40)/ITA(20)/NIPAAM(20)/TMA-BF₄(14)/ DMA-C₁₆Br(4)/SiMac(2); 19.7% Solids |
| Example 11: | AA(40)/ITA(20)/F127(20)/TMA-BF₄(18)/SiMac(2); 19.0% Solids |
| Example 12: | AA(10)/ITA(10)/NIPAAM(60)/TMA-BF₄(20); 15.2% Solids |
| Example 13: | AA(20)/ITA(20)/NIPAAM(20)/NVP(20)/TMA-BF4(20); 17.0% Solids |

### Examples 12 and 13 are reference examples.

### EXAMPLES 14 -16

### Polymers Derivatized with IEM

AA (40 parts), ITA (20 parts), NIPAAM (20 parts), TMA-BF₄ (18 parts), ODA (2 parts), VAZO-65 (1 part), and THF (300 parts) were combined in a reaction vessel and the resulting mixture purged with nitrogen for 2 minutes. The vessel was sealed and maintained at 65°C in a constant temperature rotating device for 18 hours during which time a white solid precipitate formed. After cooling to room temperature, DMF (30 parts) was added to the mixture to dissolve the solid and to the resulting solution was added IEM (1.1 parts), DBTDL (0.04 parts), and BHT (0.001 parts). After heating at 40°C for 1 hour, the resulting polymer solution was poured into a large excess of ethyl acetate and the precipitated solid collected by filtration. The solid was washed two times with ethyl acetate, dried, and dissolved in DI water to make a 22.5% solids solution that was designated Example 14 and identified as the polymer of AA (40), ITA (20), NIPAAM (20), TMAEMA-BF₄ (18), and ODA (2) with weight ratios indicated in parentheses and with a plurality (approximately ten mole percent) of the carboxy acid units derivatized with IEM to provide monomeric units of the following structure (Me = CH₃):
CH₂=C(Me)CO₂CH₂CH₂NHC(O)-Polymer backbone.

Examples 15 and 16 were prepared in a similar manner and are listed below.

| | |
|---|---|
| Example 15: | AA(40)/ITA(20)/NIPAAM(20)/MeFBSEMA(1)TMA-BF₄(19)/~IEM; 12.8% Solids |
| Example 16: | AA(40)/ITA(20)/NIPAAM(20)/MeFBSEMA(5)TMA-BF₄(15)/~IEM; 21.8% Solids |

### EXAMPLES 17-24

### Preparation of Poly(NIPAAM(55)/AA(20)/IBMA(20)/LA(5)) in Isopropanol

NIPAAM (55 parts), AA (20 parts), IBMA (20 parts), LA (5 parts), VAZO-67 (1.0 part), and isopropanol (200 parts) were combined in a reaction vessel and the resulting mixture purged with nitrogen for 2 minutes. The vessel was sealed and maintained at 65°C in a constant temperature rotating device for 18 hours during which time a clear viscous polymer solution formed. A second charge of VAZO-67 (0.20 parts) was added to the reaction vessel and the solution heated at 65°C for an additional 8 hours. After cooling to room temperature, the resulting polymer solution was poured into a large excess of ethyl acetate and the precipitated solid polymer collected by filtration. The solid was washed two times with ethyl acetate and dried in a vacuum oven at 40°C. The dried solid was designated Example 17 and identified as the polymer of NIPAAM (55), AA (20), IBMA (20), and LA (5) with weight ratios indicated in parentheses.

Dried polymers designated Examples 18-24 were prepared as described for Example 17 and are listed as follows with monomeric units and weight ratios indicated:

| | |
|---|---|
| Example 18: | NIPAAM(45), AA(20), ITA(10), IBMA(20), LA(5) |
| Example 19: | NIPAAM(45), AA(30), IBMA(20), SiMac(5) |
| Example 20: | NIPAAM(35), AA(30), ITA(10), IBMA(25) |
| Example 21: | NIPAAM(55), AA(20), IBMA(20), SiMac(5) |
| Example 22: | NIPAAM(20), AA(50), IBMA(25), SiMac(5) |
| Example 23: | NIPAAM(60), AA(20), IBMA(20) |
| Example 24: | NIPAAM(55), AA(20), IBMA(20), LA(5) |

Examples 20 and 23 are reference examples.

### EVALUATIONS

### Evaluation of Polymer Retention Times on Tooth Surfaces

To the polymer solutions designated Examples 2-5 and 21 was added 0.014% by weight of a red pigment (D&C Red 30 Talc Lake Sensient code: K7094, Sensient Technologies, St. Louis, MO) to provide Samples A-D listed in Table 1. Similarly, the red pigment was added to the product available under the trade designation SIMPLY WHITE (Colgate, New York, NY) to provide Control Sample A (CR-A). These samples were then evaluated for retention on teeth by using the following procedure.

Five extracted bovine teeth were removed from their storage container filled with water, and briefly patted with a paper towel. Samples A-D and CR-A were then individually brushed on each tooth and after 1 minute, the coated teeth were immersed in a water bath that was maintained at 37°C and continuously agitated. The teeth were visually monitored over 1 hour for changes in the intensity and continuity of the red color on the tooth. The period of time that the red-colored polymer coating remained on a tooth was reported as the "Retention Time". After 1 hour, the coated teeth were removed from the water bath and probed gently to gauge the integrity of the tooth coatings. Results are provided in Table 1. Overall, based on direct observations of color intensity and continuity, Samples A-D all appeared to maintain a retentive film for greater than 1 hour, whereas Sample CR-A lost substantially all of the red coating color within 5 minutes.

| Table 1 | | | |
|---|---|---|---|
| Sample | Composition | Retention Time | Observations |
| A | Ex. 2 + red pigment | >1 hour | Coating was retained but fragile to the touch (loss of red color and coating fragmented when probed) |
| B | Ex. 3 + red pigment | >1 hour | Coating less fragile then Samples B and D (more difficult to remove red color and coating with probe) |
| C | Ex. 4 + red pigment | >1 hour | Coating was retained but fragile to the touch (loss of red color and coating fragmented when probed) |
| D | Ex. 5 + red pigment | >1 hour | Least fragile coating (most difficult coating to remove red color and fragment coating with probe) |
| E | Ex. 21 | > 1 hour | Coating was retained |
| CR-A | "SIMPLY WHITE" + red pigment | <5 5 minutes | Color loss began immediately when coated tooth placed in water bath. |

### Evaluation of Polymers/Whitening Agent on Tooth Surfaces

To the polymer solutions designated Examples 2-5 was added 18% by weight carbamide peroxide (Sigma-Aldrich) to provide Samples E-H listed in Table 2. These Samples were then evaluated for whitening of teeth by using the following procedure.

Eight extracted bovine teeth were removed from their storage container filled with water and stained by soaking in a solution of Classic Coke and coffee for 24 hours at 38°C. An initial determination of tooth shade was determined for each tooth by comparing the shade with a VITA Shade Guide (Vident, Brea, CA). Samples E-H were then individually painted on the surface of each tooth with a brush and air dried for 30 seconds. Two teeth were identically coated with each polymer solution sample. The coated teeth were immersed in a water bath that was maintained at 37°C and continuously agitated. After 30-45 minutes, the coated teeth were removed from the water bath and coated again with the samples in a similar manner. This procedure was repeated until the teeth had been coated a total of 10 times. The teeth were then removed from the water bath and a final determination of tooth shade was determined for each tooth by comparing the shade with the VITA Shade Guide. The differences between the initial and the final stain values were calculated with a larger difference being indicative of increased tooth whitening. Results as an average of the two replicates per sample are provided in Table 2. All samples showed an increase in teeth whitening when evaluated under the conditions of this test method.

| Table 2 | | |
|---|---|---|
| Sample | Composition | Change in Stain Value (Increase in Whitening) |
| E | Ex. 2 + carbamide peroxide | 3.5 |
| F | Ex. 3 + carbamide peroxide | 2.5 |
| G | Ex. 4 + carbamide peroxide | 4.0 |
| H | Ex. 5 + carbamide peroxide | 3.5 |

## Claims

1. A dental whitening composition suitable for coating oral surfaces comprising:
a tooth whitening agent; and
a polymer comprising:
a repeating unit comprising a polar or polarizable group; and
a repeating unit comprising a group selected from the group consisting of a hydrophobic hydrocarbon group having a molecular weight of at least 160, a graft polysiloxane chain, a hydrophobic fluorine-containing group, and combinations thereof,
with the proviso that the polymer does not include pendant ethylenically unsaturated moieties.

2. The dental whitening composition according to claim 1, wherein the polymer further comprises a repeating unit comprising a fluoride releasing group.

3. The dental whitening composition of claim 1 or 2, wherein the tooth whitening agent is selected from the group consisting of a hypochlorite, an organic peroxide, an inorganic peroxide, a hydroperoxide, a peracid, carbamide peroxide, and combinations thereof.

4. The dental whitening composition according to claim 1, wherein the composition comprises at least 0.5% by weight of the tooth whitening agent, based on the total weight of the tooth whitening agent and polymer.

5. The dental whitening composition according to any of the preceding claims, wherein the polymer further comprises a repeating unit comprising a modulating group.

6. The dental whitening composition according to any of the preceding claims, wherein the repeating unit comprising the polar or polarizable group is selected from the group consisting of acrylic acid, itaconic acid, N-isopropylacrylamide, and combinations thereof.

## Patentansprüche

1. Zahnaufhellungszusammensetzung, die zum Beschichten von oralen Oberflächen geeignet ist, umfassend:
ein Zahnaufhellungsmittel und
ein Polymer, umfassend:
eine Wiederholungseinheit mit einer polaren oder polarisierbaren Gruppe und
eine Wiederholungseinheit mit einer Gruppe aus der Gruppe bestehend aus einer hydrophoben Kohlenwasserstoffgruppe mit einem Molekulargewicht von mindestens 160, einer Pfropfpolysiloxankette,
einer hydrophoben fluorhaltigen Gruppe und Kombinationen davon,
mit der Maßgabe, daß das Polymer keine seitenständigen ethylenisch ungesättigten Gruppierungen enthält.

2. Zahnaufhellungszusammensetzung nach Anspruch 1, wobei das Polymer ferner eine Wiederholungseinheit mit einer fluoridabgebenden Gruppe umfaßt.

3. Zahnaufhellungszusammensetzung nach Anspruch 1 oder 2, wobei das Zahnaufhellungsmittel aus der Gruppe bestehend aus einem Hypochlorit, einem organischen Peroxid, einem anorganischen Peroxid, einem Hydroperoxid, einer Persäure, Carbamidperoxid und Kombinationen davon ausgewählt ist.

4. Zahnaufhellungszusammensetzung nach Anspruch 1, wobei die Zusammensetzung mindestens 0,5 Gew.-% des Zahnaufhellungsmittels, bezogen auf das Gesamtgewicht des Zahnaufhellungsmittels und des Polymers, umfaßt.

5. Zahnaufhellungszusammensetzung nach einem der vorhergehenden Ansprüche, wobei das Polymer ferner eine Wiederholungseinheit mit einer modulierenden Gruppe umfaßt.

6. Zahnaufhellungszusammensetzung nach einem der vorhergehenden Ansprüche, wobei die Wiederholungseinheit mit der polaren oder polarisierbaren Gruppe aus der Gruppe bestehend aus Acrylsäure, Itaconsäure, N-Isopropylacrylamid und Kombinationen davon ausgewählt ist.

## Revendications

1. Composition de blanchiment des dents convenable pour le recouvrement de surfaces orales comprenant :
un agent de blanchiment des dents ; et
un polymère comprenant :
un motif de répétition comprenant un groupement polaire ou polarisable ; et
un motif de répétition comprenant un groupement choisi dans le groupe constitué d'un groupement hydrocarboné hydrophobe ayant un poids moléculaire d'au moins 160, une chaîne polysiloxane greffée, un groupement fluoré hydrophobe, et des combinaisons de ceux-ci,
à condition que le polymère ne comporte pas de motifs éthyléniquement insaturés pendants.

2. Composition de blanchiment des dents selon la revendication 1, **caractérisée en ce que** le polymère comprend en outre un motif de répétition comprenant un groupement de libération de fluorure.

3. Composition de blanchiment des dents selon la revendication 1 ou 2, **caractérisée en ce que** l'agent de blanchiment des dents est choisi dans le groupe constitué d'un hypochlorite, un peroxyde organique, un peroxyde minéral, un hydroperoxyde, un peracide, un peroxyde de carbamide, et des combinaisons de ceux-ci.

4. Composition de blanchiment des dents selon la revendication 1, **caractérisée en ce que** la composition comprend au moins 0,5 % en poids d'agent de blanchiment des dents, sur la base du poids total de l'agent de blanchiment des dents et du polymère.

5. Composition de blanchiment des dents selon l'une quelconque des revendications précédentes, **caractérisée en ce que** le polymère comprend en outre un motif de répétition comprenant un groupement modulateur.

6. Composition de blanchiment des dents selon l'une quelconque des revendications précédentes, **caractérisée en ce que** le motif de répétition comprenant le groupement polaire ou polarisable est choisi dans le groupe constitué de'acide acrylique, de'acide itaconique, du N-isopropylacrylamide, et des combinaisons de ceux-ci.
